# EUROPEAN PATENT SPECIFICATION

(11) **EP 2 630 941 B1**
(45) Date of publication and mention of the grant of the patent: **30.05.2018**
(21) Application number: 11834438.1
(22) Date of filing: 20.10.2011
(51) Int. Cl.: A61G 7/05, A61B 5/11, G08B 21/22

(54) **DEVICE FOR ASCERTAINING IF PATIENTS GET OUT OF BED**
VORRICHTUNG ZUR FESTSTELLUNG, OB EIN PATIENT SEIN BETT VERLASSEN HAT
DISPOSITIF POUR DÉTERMINER SI DES PATIENTS SORTENT OU NON DU LIT

(30) Priority: 20.10.2010 JP 2010235102
(43) Date of publication of application: 28.08.2013
(73) Proprietor: Sanin Seigyo Corporation, Yasugi-shi, Shimane 692-0011 (JP)
(72) Inventor: HANADA Eisuke, Izumo-shi Shimane 6938501 (JP); HATA Hiroshi, Yasugi-shi Shimane 692-0011 (JP)
(74) Representative: Müller-Boré & Partner Patentanwälte PartG mbB
(86) International application number: PCT/JP2011/074170
(87) International publication number: WO 2012/053599

(56) References cited:
- JP-A- 2001 000 401
- JP-A- 2007 014 725
- JP-A- 2008 067 979
- US-A1- 2005 124 864
- US-A1- 2007 163 045
- US-A1- 2008 136 226
- US-A1- 2009 237 264

## Description

### Technical Field

The present invention relates to a device for checking getting out of bed to easily detect the presence or absence of a person or change of the body position of the person on a bed easily.

### Background Art

A patient who should stay in bed in a hospital and the like may get up or leave from a bed. Various devices for checking getting out of bed have been developed to transmit such getting out of bed promptly to a nurse station, for example. The devices for checking out of bed are used effectively in detecting wandering to occur at health care facilities for elderly persons, for example.

Many of these devices for checking getting out of bed use a piezoelectric element as a sensor. Examples of these devices include a device in which a sensor is placed on a floor beside a bed (Patent Literature 1), a device in which a sensor is placed on a bed mat (Patent Literature 2), and a device in which a sensor is attached inside a mattress (Patent Literature 3).

### Citation List

### Patent Literatures

[Patent Literature 1] Japanese Patent Application Laid-Open No. 2003-159287
[Patent Literature 2] Japanese Patent Application Laid-Open No. Hei. 8-80286
[Patent Literature 3] Japanese Patent Application Laid-Open No. 2005-237684

### Summary of Invention

### Technical Problem

If a sensor is placed on a floor beside a bed (Patent Literature 1), however, the sensor cannot detect getting up of a patient. Further, the sensor on the floor beside the bed could be a cause of falling of the patient when the patient gets down from the bed. If a sensor is placed on a bed mat (Patent Literature 2), the sensor makes a bed uncomfortable. If a sensor is attached inside a mattress (Patent Literature 3), the sensitivity of the sensor changes widely depending on the performance of the mattress in distributing a weight, for example. Thus, in some cases, an expected function cannot be achieved.

Additionally, if a piezoelectric element is used as a sensor of a device for checking getting out of bed, detection sensitivity is hard to control. Further, the device cannot be manufactured at low cost, and maintenance thereof entails high cost. Meanwhile, if a pressure-sensitive sensor in the form of a sheet is used, the pressure-sensitive sensor should be fixed to a mattress by sewing the sensor on a mattress, for example. Thus, even attachment of the device for checking getting out of bed to a bed is difficult. This prevents widespread use of a device for checking getting out of bed, although the usefulness of the device has been recognized in the fields of hospitals and nursing care.

US 2009/237264 A1 discloses a control unit for a patient support, such as a mattress. The control unit comprises a pad including head sensors and seat sensors which enable the control unit to detect presence and absence of a patient lying on the mattress, to detect a lack of patient movement over a period of time and to distinguish between different positions of the patient lying on the mattress. The pad with the head and seat sensors is arranged between a bladder assembly and a filler portion positioned substantially underneath the pad and the pad being positioned underneath the bladder assembly. A sensory algorithm as well as a control algorithm enable the control unit to detect various positions of the patient lying on the mattress and also to measure the patients weight within ± 20%.

US 2008/136226 A1 discloses an apparatus for detecting the presence or absence of a person on a flexible support, such as a seat or a bed. The respective apparatus includes a sensor which indicates when tension of the assembly exceeds a certain amount which is indicative of sagging of the support. The sensor is a switch that includes a plunger which operates against the bias of a biasing member which either completes or interrupts an electrical circuit in response to tension placed on the assembly due to sagging of the support.

In response to the foregoing, it is an object of the present invention to provide a device for checking getting out of bed capable of reliably detecting the presence or absence or change of a body position, and which is usable in determination about care to prevent bedsores and about check of living as a result of detection of an interval of the change of the body position, available at low cost, easy to attach to and detach from a bed, and easy to perform maintenance.

### Solution to Problem

The inventors have found that the aforementioned object is achieved by using a tactile switch, which is applied to a keyboard of a personal computer and the like, as a sensor part of a device for checking getting out of bed, and by arranging the tactile switch in a specific gap formed below a mattress by an elastic member that biases the lower surface of the mattress upward.

To be specific, the present invention provides a device for detecting the presence/absence of a person in a bed according to claim 1.

Preferably , the bed includes a plurality of elastic members that are provided at least at a central part of the floor material of the bed biasing the lower surface of the mattress upward.

Preferably, the elastic member is disposed on the floor material of the bed and biases the lower surface of the mattress upward, or is attached to the lower surface of the mattress and biases the lower surface of the mattress upward.

### Advantageous Effects of Invention

According to the device for checking getting out of bed of the present invention, the plurality of tactile switches is disposed in the specific gap formed below the mattress by the elastic members, and the controller detects the presence or absence of a person and/or change of the body position of the person on the mattress on the basis of ON or OFF of the tactile switches, preferably, also detects an interval of the change of the body position. Thus, the device of the present invention is usable to check getting out of bed, prevention of bedsores, check of living and the like.

The tactile switches can be obtained at low cost, and can be exchanged easily during maintenance of the device.

In particular, the device can be attached easily to an existing bed with a gap formed below a mattress by an elastic member provided to the bed, and can be maintained easily.

### Brief Description of the Drawings

Fig. 1 shows the entire structure of an embodiment of a first aspect of a device for checking getting out of bed.
Fig. 2A is a rear view of a strip-shaped sensor.
Fig. 2B is a cross-sectional view of the strip-shaped sensor.
Fig. 3A is a plan view of a cross beam with
   butterfly springs.
Fig. 3B is a cross-sectional view of the cross beam with the butterfly spring.
Fig. 4 is a cross-sectional view showing the butterfly spring in a condition where a mattress is placed on the butterfly spring.
Fig. 5A is a plan view showing a condition where the strip-shaped sensor is attached to the cross beam with the butterfly springs.
Fig. 5B is a cross-sectional view showing the condition where the strip-shaped sensor is attached to the cross beam with the butterfly spring.
Fig. 6A is an explanatory view of the operation of the embodiment implemented by attachment of the device for checking getting out of bed.
Fig. 6B is an explanatory view of the operation of the embodiment implemented by attachment of the device for checking getting out of bed.
Fig. 7A is a perspective view of part of a strip-shaped sensor of a modification of the embodiment of the first aspect.
Fig. 7B is a cross-sectional view showing a condition where the strip-shaped sensor of the modification of the embodiment of the first aspect is attached to a cross beam of a bed.
Fig. 8 is a perspective view of part of a strip-shaped sensor of an embodiment of a second aspect of the device for checking getting out of bed.
Fig. 9A is a cross-sectional view showing a condition where the strip-shaped sensor of the embodiment of the second aspect is attached to a floor material of a bed.
Fig. 9B is an explanatory view of the operation of the strip-shaped sensor of the embodiment of the second aspect.
Fig. 10A is a cross-sectional view showing a condition where the strip-shaped sensor of the embodiment of the second aspect is attached to the lower surface of a mattress.
Fig. 10B is an explanatory view of the operation of the strip-shaped sensor of the embodiment of the second aspect.

### Description of Embodiments

A device for checking getting out of bed of the present invention will be described in detail below on the basis of the drawings. The same or corresponding constituent elements in the drawings are denoted by the same reference numerals.

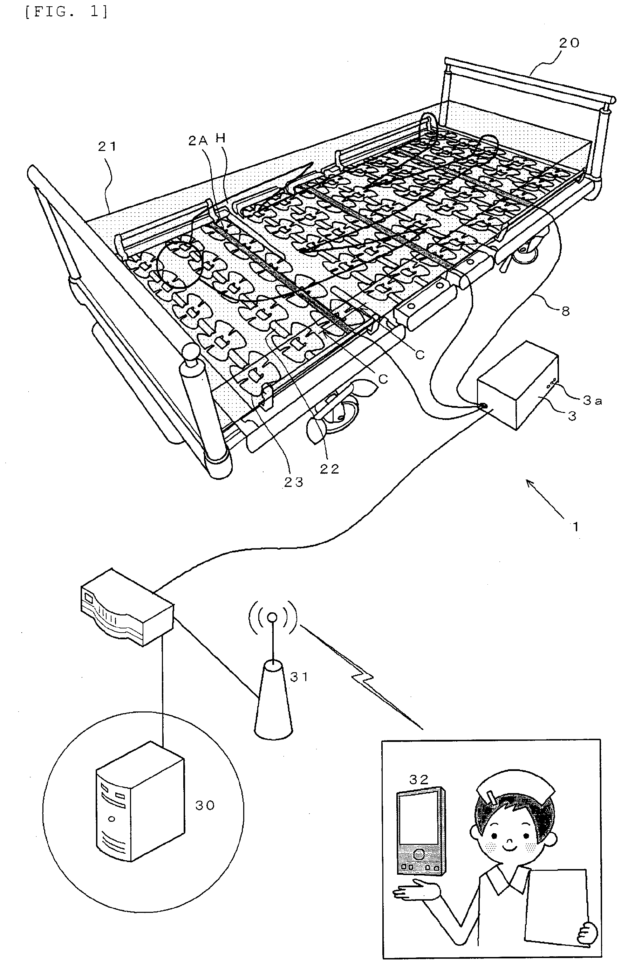

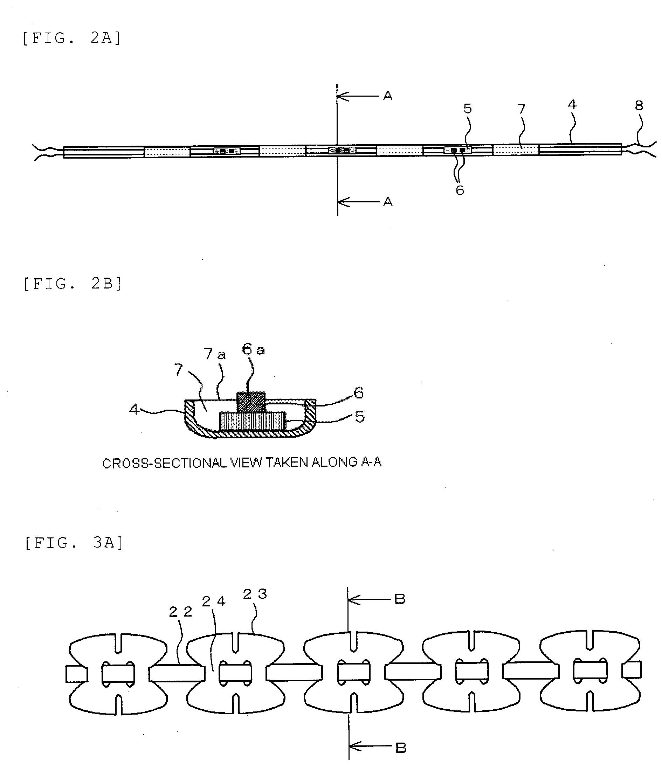

The device for checking getting out of bed of the present invention includes a device for checking getting out of bed of a first aspect having no elastic member that biases the lower surface of a mattress upward. Fig. 1 shows an embodiment of the device for checking getting out of bed of the first aspect attached to a bed 20. Fig. 2A is a rear view of a strip-shaped sensor 2A constituting the device 1 for checking getting out of bed of Fig. 1. Fig. 2B is a cross-sectional view taken along A-A of Fig. 2A.

As shown in Fig. 1, the device 1 for checking getting out of bed includes three strip-shaped sensors 2A, and a controller 3 connected through cables 8 to the strip-shaped sensors 2A.

As shown in Figs. 2A and 2B, the strip-shaped sensors 2A each include a strip-shaped flexible plastic substrate 4 curved into a U-shape in cross section, and tactile switch mounting substrates 5 and attachment position control members 7 for the strip-shaped sensor 2A provided at certain intervals to the plastic substrate 4. Two tactile switches 6 are mounted so as to be close to each other on each of the tactile switch mounting substrates 5.

Here, the tactile switches 6 are switches, each having a contact, that are turned ON and OFF by a force applied to a plunger at a tip end portion thereof and deformation of an inner spring. The tactile switches 6 are different from a touch switch that is turned ON and OFF only by touch of a tip end portion thereof. Using such switches with contacts as sensors of the device for checking getting out of bed makes it possible to achieve a very simple structure of the device for checking getting out of bed in terms of an electrical aspect. Thus, the device for checking getting out of bed can be manufactured at low cost, and a failure of the device can be handled easily.

The tactile switches 6 are selected from commercially available switches of various types requiring different forces for making the switches ON and OFF, and having different outer shapes and sizes, for example. The tactile switches 6 can be selected suitably depending on a bed on which the device for checking getting out of bed is to be installed. It is preferable, for example, to use a tactile switch (tactile switches of standard type B3F series, available from OMRON Corporation, for example) that is turned ON in response to a pressing force of from 0.9 to 5 N and conventionally used for a keyboard of a personal computer and the like.

In the strip-shaped sensors 2A, the positions of end surfaces 6a of the tactile switches 6 and the positions of end surfaces 7a of the attachment position control members 7 are adjusted as follows with respect to the bed 20 to which the strip-shaped sensors 2A are to be attached.

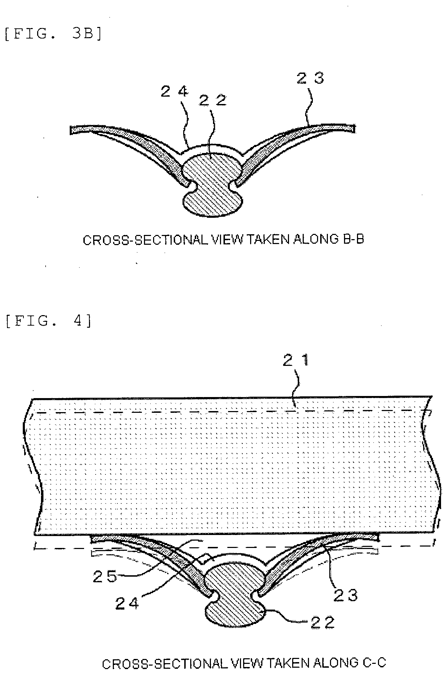

To be specific, the bed 20 is configured such that, in order to prevent bedsores of a user of the bed, a plurality of butterfly springs 23 are attached to cross beams 22 with butterfly spring fitting parts 24, a mattress 21 is placed on the butterfly springs 23, so that the lower surface of the mattress 21 is biased upward by the butterfly springs 23. Fig. 3A is a plan view of the cross beam 22 with the butterfly springs 23. Fig. 3B is a cross-sectional view taken along B-B of Fig. 3A. Fig. 4 is a cross-sectional view taken along C-C (Fig. 1), and showing the butterfly spring 23 in a condition where the mattress 21 is placed on the butterfly spring 23. As shown by solid lines of Fig. 4, in the bed 20, a gap 25 is formed below the mattress 21 by the presence of the butterfly spring 23, and the dimension of the gap 25 is changed by the presence or absence of a person on the mattress 21. More specifically, if a person is present on the mattress 21, the butterfly spring 23 expands as shown by dashed lines of Fig. 4 to reduce the gap 25 below the mattress 21. At this time, the bed user can receive appropriate stimulation by the butterfly spring 23. Commercially available beds are applicable as the bed 20 with the butterfly springs 23. For example, a bed having a function of micro-stimulation, available from Völker GmbH, is applicable as the bed 20.

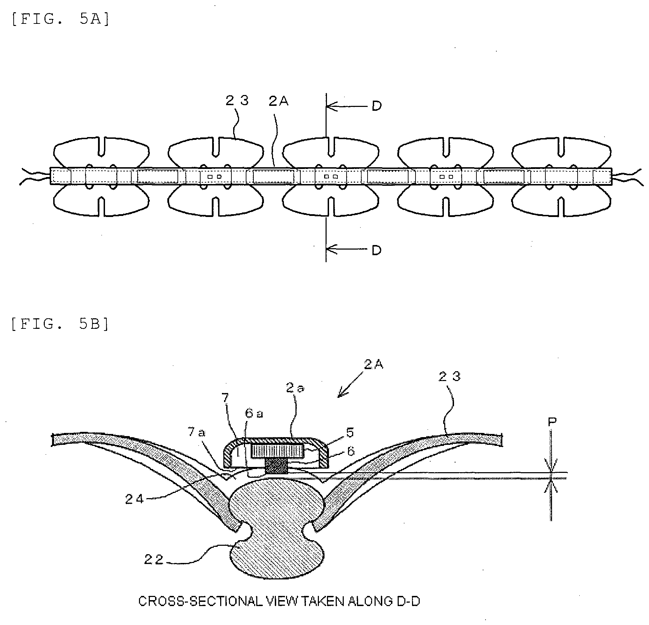

In this embodiment, as shown in Fig. 1, the strip-shaped sensors 2A are attached to the cross beams 22 to hold the mattress 21 of the bed 20 thereon such that the tactile switches 6 face the upper surfaces of the cross beams 22. Fig. 5A is a plan view showing a condition where the strip-shaped sensor 2A is put on the cross beam 22 with the butterfly springs 23. Fig. 5B is a cross-sectional view taken along D-D of Fig. 5A. While the strip-shaped sensor 2A is attached to the cross beam 22, the end surface 7a of the attachment position control member 7 makes abutting contact with the upper surface of the butterfly spring fitting part 24 engaging with the cross beam 22. With the strip-shaped sensor 2A attached to the cross beam 22, the position of the end surface 7a of the attachment position control member 7 of the strip-shaped sensor 2A is determined such that, if a person is not present on the mattress 21, an upper surface 2a of the strip-shaped sensor 2A is not in contact with a lower surface 21a of the mattress 21, so that a slight void P is formed between the end surface 6a of the tactile switch 6 and a surface of the cross beam 22. The position of the end surface 7a is also determined such that, if a person is present on the mattress 21, the butterfly spring 23 expands to make the lower surface 21a of the descending mattress 21 press the upper surface 2a of the strip-shaped sensor 2A, thereby making the surface of the cross beam 22 press the end surface 6a of the tactile switch 6.

The controller 3 includes computing means such as a PLC (programmable logic controller), and is connected to all the tactile switches 6. The computing means of the controller 3 is programmed so as to detect the presence or absence of a person or change of the body position of the person on the mattress 21 in response to ON or OFF of the tactile switches 6, and to output a signal indicating getting out of bed if detecting the absence. As an example, the controller 3 outputs the signal indicating getting out of bed through an output terminal 3a of the controller 3 if all the tactile switches 6 are OFF. Further, the controller 3 stores positions where the tactile switches 6 are attached to the bed 20, and detects an area where the tactile switches 6 are ON, thereby detecting an area where a weight is applied onto the mattress 21. As a result, the controller 3 detects change of a body position and outputs the change. More specifically, if only the tactile switches 6 on one side of the bed are ON, it is considered that a body has moved to a position where the tactile switches 6 are ON. In this case, the controller 3 outputs the change of the body position through the output terminal 3a.

It is preferable that the controller 3 store intervals between ON and OFF of the tactile switches 6 (specifically, interval of change of a body position), and output a signal urging care to prevent bedsores or a signal indicating necessity of check of living if the intervals are longer than a fixed period of time.

Communication means may be connected to the controller 3. In this case, a signal from the controller 3 indicating the presence or absence of a person or change of the body position of the person on the mattress 21, necessity of care to prevent bedsores, necessity of check of living and the like may be transmitted from the communication means through a public telephone network, the Internet, or a wired or wireless LAN to a terminal of a nurse station or a portable phone of a nurse or a caregiver, for example. As an example, the controller 3 is connected through a wired or wireless network to an access point 31 of the Internet or an on-site server 30, and a signal output from the controller 3 is transmitted by communication radio waves of a wireless LAN and the like to a terminal 32 used by a nurse.

The device 1 for checking getting out of bed shown in Fig. 1 is used as follows.

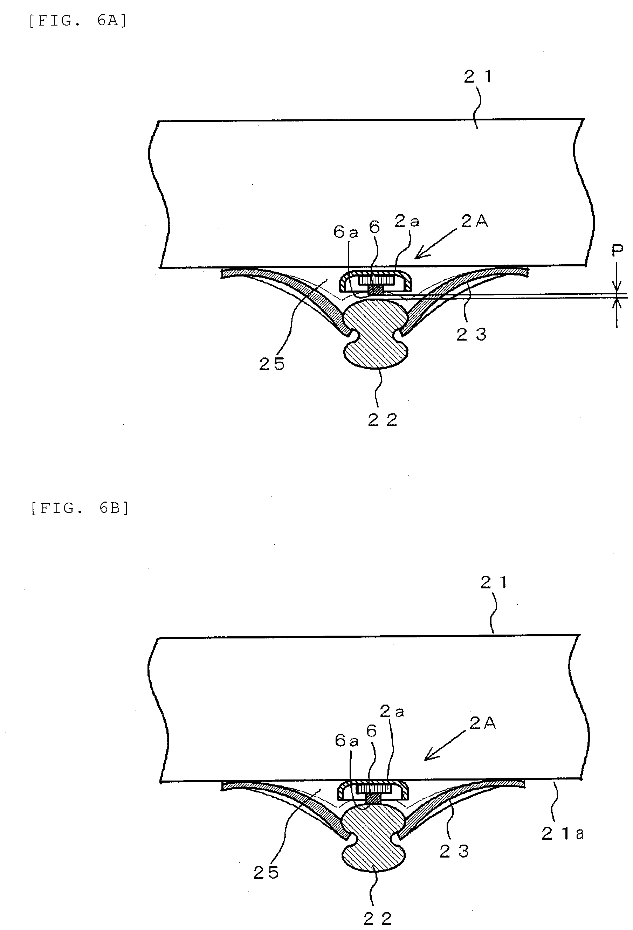

First, the three strip-shaped sensors 2A are attached to the cross beams 22 with the butterfly springs 23 such that, while a person lies on at least a central part of the sleeping surface of the bed 20, namely, while a person lies in bed, the tactile switches 6 are arranged at positions corresponding to an area where the bed 20 is pressed. More specifically, as shown in Fig. 1, the strip-shaped sensors 2A are attached to the cross beams 22 with the butterfly springs 23 such that the tactile switches 6 are arranged at positions corresponding to the shoulder, waist, and legs of a person H lying on the mattress 21. As shown in Fig. 6A, if a person is not present on the mattress 21, the strip-shaped sensor 2A with the tactile switch 6 is within the gap 25 formed directly below the mattress 21 by the butterfly spring 23, so that the sensor 2A is not in contact with the mattress 21. Thus, the slight space P is formed between the end surface 6a of the tactile switch 6 and a surface of the cross beam 22, so that no pressing force is applied to the tactile switch 6. As a result, the tactile switch 6 becomes OFF. In contrast, as shown in Fig. 6B, if a person is present on the mattress 21, the butterfly spring 23 expands to lower the lower surface 21a of the mattress 21. This presses the upper surface 2a of the strip-shaped sensor 2A, so that the end surface 6a of the tactile switch 6 is pressed by the surface of the cross beam 22. As a result, the tactile switch 6 becomes ON.

For example, if all the tactile switches 6 of the device 1 for checking getting out of bed are OFF, a signal indicating getting out of bed is output from the controller 3. As a result, person's getting out of the bed 20 can be detected reliably.

After each of the tactile switches 6 is turned ON once in response to the weight of a bed user on the mattress 21, the tactile switch 6 is turned OFF if the weight is removed. The tactile switches 6 are repeatedly turned ON and OFF precisely in response to the presence or absence of a weight. Thus, the device 1 for checking getting out of bed is capable of detecting the presence or absence of getting out of bed continuously with high reliability.

Regarding installation of the device 1 for checking getting out of bed on the bed 20, the positions on the cross beam 22 to receive the strip-shaped sensor 2A can be changed appropriately depending on a purpose of detection.

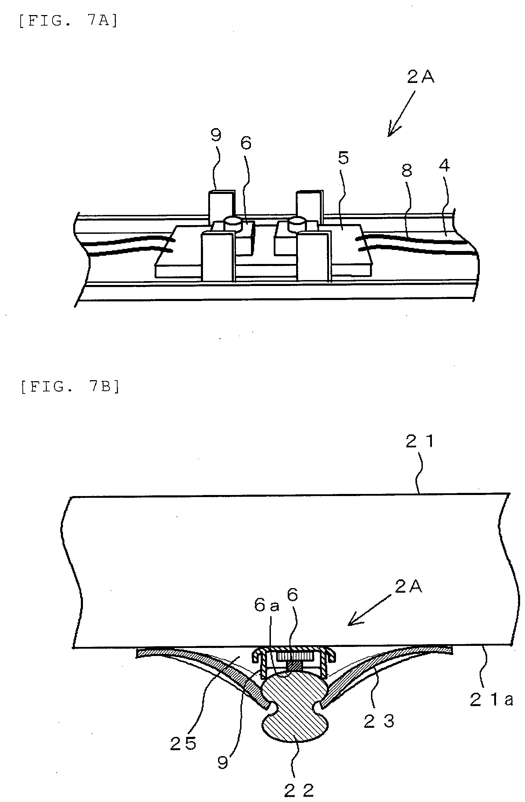

The first aspect of the device for checking getting out of bed of the invention has been described above based on the embodiment shown in Fig. 1. Meanwhile, various aspects of the device for checking getting out of bed of the present invention can be devised. For example, it is preferable that, in the aforementioned device 1 for checking getting out of bed, plate-like strips 9 provided at opposite outer sides of the tactile switch mounting substrate 5 project from the strip-shaped plastic substrate 4 to a height greater than the end surfaces 6a of the tactile switches 6 as shown in Fig. 7A. In this case, as shown in Fig. 7B, the projection height of the plate-like strips 9 from the strip-shaped plastic substrate 4 is determined such that, while the butterfly spring 23 expands in response to the weight of a person on the mattress 21 to lower the lower surface 21a of the mattress 21, so that the strip-shaped sensor 2A is pressed and the end surface 6a of the tactile switch 6 is pressed by the surface of the cross beam 22, the plate-like strips 9 make abutting contact with the surface of the cross beam 22. This makes it possible to prevent application of excessive pressing force on the tactile switches 6, so that the durability of the strip-shaped sensors 2A is enhanced. This also makes it possible to prevent turning ON of the tactile switches 6 erroneously to occur if some weight is applied on the mattress 21 even in the absence of a person on the mattress 21.

A bed to which the device 1 for checking getting out of bed is attached is not limited to the aforementioned bed 20 provided with the cross beams 22 with the butterfly springs 23. The device is also applicable to a bed, as long as the bed includes an elastic member that biases the lower surface of a mattress upward from a floor material such as a beam and a flat plate to hold the mattress thereon, and the elastic member forms a gap directly below the lower surface of the mattress.

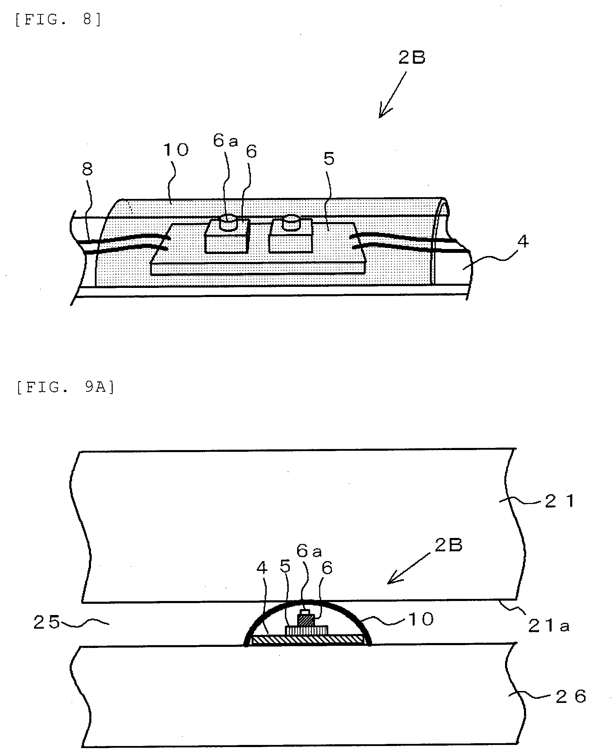

Fig. 8 is a perspective view of part where tactile switches are mounted on a strip-shaped sensor 2B of a device for checking getting out of bed of an embodiment of the second aspect of the present invention. The strip-shaped sensor 2B of this device for checking getting out of bed differs from the strip-shaped sensors 2A of the
device 1 for checking getting out of bed of Fig. 1 in that the sensor 2B includes an elastic member that is placed on a floor material such as a beam and a flat plate to hold a mattress directly thereon and biases the lower surface 21a of the mattress 21 upward. More specifically, the sensor 2B includes a resin elastic member 10 as this elastic member.

The resin elastic member 10 has the gutter arcuate cross section. As shown in Fig. 8, the resin elastic member 10 is formed into a size that makes the resin elastic member 10 cover at least the tactile switch mounting substrate 5 when the resin elastic member 10 is put on the strip-shaped sensor 2B, and makes the resin elastic member 10 not to be in contact with the end surfaces 6a of the tactile switches 6 mounted on the tactile switch mounting substrate 5 if the resin elastic member 10 is not pressed.

As an example, as shown in Fig. 9A, the strip-shaped sensor 2B with the resin elastic member 10 is placed on a floor material 26 of a bed such that the top of the arc of the resin elastic member 10 is pointed toward the mattress 21, and the mattress 21 is placed on the resin elastic member 10. As a result, the gap 25 is formed between the mattress 21 and the floor material 26.

Lower edge portions of the resin elastic member 10 are not fixed to the strip-shaped plastic substrate 4 while the resin elastic member 10 is attached to the floor material 26 of the bed. Thus, if the resin elastic member 10 is pressed in a direction in which the arc is flattened out, the lower edge portions thereof move farther toward the outer side of the strip-shaped plastic substrate 4. As the lower edge portions of the resin elastic member 10 move toward the outer side in response to pressure application, the resin elastic member 10 can be deformed easily.

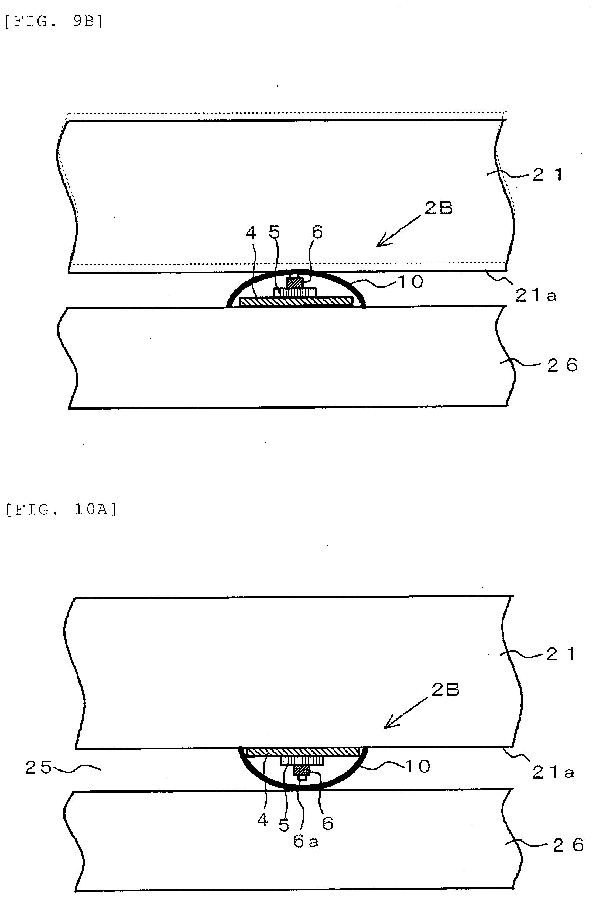

Regarding the elasticity of the resin elastic member 10, the resin constitution, the thickness and the like of the resin elastic member 10 are selected appropriately to achieve the following. With the strip-shaped sensor 2B placed between the mattress 21 and the floor material 26 as shown in Fig. 9A, if a person is present on the mattress 21, the resin elastic member 10 is flatted out to make the resin elastic member 10 be in contact with the end surface 6a of the tactile switch 6, thereby turning the tactile switch 6 ON as shown in Fig. 9B. Meanwhile, if a person is not present on the mattress 21, the resin elastic member 10 maintains its arcuate cross section so that the tactile switch 6 is not turned ON.

Thus, like in the case of the aforementioned device for checking getting out of bed of the first aspect, person's getting out of bed can be detected reliably by placing the strip-shaped sensor 2B with the resin elastic member 10 on the floor material 26 of the bed to hold the mattress 21 thereover and placing the mattress 21 on the strip-shaped sensor 2B.

Fig. 10A shows a condition where the strip-shaped plastic substrate 4 of the strip-shaped sensor 2B with the resin elastic member 10 is attached to the lower surface of the mattress 21 with an attachment tool not shown in the drawings, and the projection of the arc of the resin elastic member is pointed toward the floor material 26. This attachment tool could be any tool with which the strip-shaped sensor 2B is attached to the lower surface of the mattress 21 such that the resin elastic member 10 does not deviate from a position where the resin elastic member 10 is put on the strip-shaped plastic substrate 4. The strip-shaped plastic substrate 4 of the strip-shaped sensor 2B may be bonded with an adhesive to the lower surface 21a of the mattress 21.

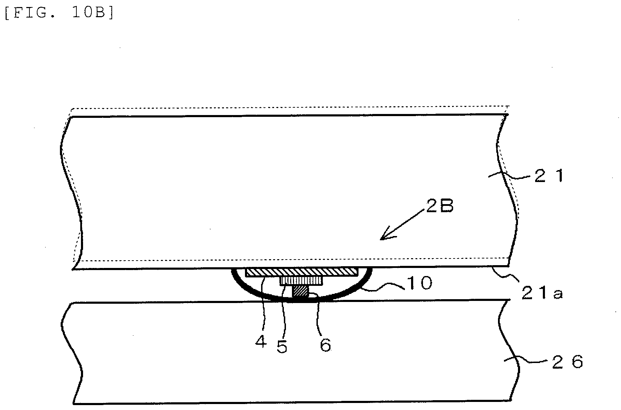

When the strip-shaped sensor 2B with the resin elastic member 10 is attached to the lower surface of the mattress 21 and the mattress 21 in this condition is placed over the floor material 26, if a person is present on the mattress 21, the resin elastic member 10 is also flatted out to make the resin elastic member 10 be in contact with the end surface 6a of the tactile switch 6 as shown in Fig. 10B, thereby turning the tactile switch 6 ON. Meanwhile, if a person is not present on the mattress 21, the resin elastic member 10 maintains its arcuate cross section so that the tactile switch 6 is not turned ON. Thus, person's getting out of bed can be detected reliably.

In the present invention, the shape of the tactile switch mounting substrates 5, the number of the tactile switches 6 mounted on the tactile switch mounting substrates 5, the respective shapes of the strip-shaped sensors 2A and 2B, the respective numbers of the tactile switch mounting substrates 5 to be attached to the strip-shaped sensors 2A and 2B, and others can be changed in various ways depending on the dimension or the shape of the gap 25 formed between the mattress 21 and the floor material 26 to hold the mattress 21 thereover and by an elastic member that biases the lower surface 21a of the mattress 21 upward, the withstand load of the tactile switches 6 or the like in the bed on which the device for checking getting out of bed is to be installed.

Determination as to if the resin elastic member 10 with the strip-shaped sensor 2B is to be placed on the floor material 26 of a bed or to be attached to the lower surface of the mattress 21 can be made appropriately depending on the structure of a floor material or the properties of the lower surface of a mattress of the bed to which the strip-shaped sensor 2B is to be attached.

Further, the resin elastic member 10 with the strip-shaped sensor 2B is not only a resin elastic member having the gutter arcuate cross section but also a spring-loaded elastic member or a plate spring elastic member. However, a resin elastic member having the shape of a gutter is a preferable member because it can be formed easily and at low cost.

Commercially available pressure distribution beds used as beds for nursing care are applicable as a bed to which the device for checking getting out of bed can be attached. A bed including a flat plate as the floor material 26 of the bed is particularly preferable.

### Reference Signs List

1 device for checking getting out of bed
2A, 2B strip-shaped sensor
2a upper surface of strip-shaped sensor
3 controller
3a output terminal of controller
4 strip-shaped plastic substrate
5 tactile switch mounting substrate
6 tactile switch
6a end surface of tactile switch
7 attachment position control member
7a end surface of attachment position control member
8 cable
9 plate-like strip
10 resin elastic member
20 bed
21 mattress
21a lower surface of mattress
22 cross beam
23 butterfly spring
24 butterfly spring fitting part
25 gap
26 floor material
30 server
31 access point
32 terminal
H person
P space

## Claims

1. A device for detecting the presence/absence of a person in a bed, comprising:
a plurality of switches (6) disposable in a gap (25) between the lower surface of a mattress (21) and a floor material (26) of a bed (20) to hold the mattress (21) thereover,
the switches (6), each having a contact, are turned ON and OFF by a force applied, and
a controller (3) that detects the presence or absence of the person and/or change of a body position of the person on the mattress (21) on the basis of ON or OFF of the switches (6),
**characterised in that**
said switches are tactile switches, each having a contact that is turned ON and OFF by a force applied to a plunger at a tip end portion thereof and by deformation of an inner spring, and
**in that** the gap is being formed by an elastic member between the lower surface of the mattress and the floor material of the bed to hold the mattress thereover, the elastic member biasing the lower surface of the mattress upward.

2. The device according to claim 1, wherein the plungers are having flat surfaces at top
ends thereof.

3. The device according to claim 1 or 2, wherein the bed (20) has a plurality of the elastic members (23) biasing the lower surface of the mattress (21) upward at least at a central part of the floor material (26) of the bed (20) to hold the mattress (21) thereover.

4. The device according to any of claims 1 to 3, wherein the tactile switches (6) are provided to a strip-shaped substrate (4) engaging with a floor or a beam.

5. The device according to claim 1 or 2, wherein the elastic member (10) is disposed on the floor material (26) of the bed (20) and biasing the lower surface of the mattress (21) upward.

6. The device according to claim 1 or 2, wherein the elastic member (10) is attached to the lower surface of the mattress (21) and biasing the lower surface of the mattress (21) upward.

7. The device according to claim 5 or 6, wherein the elastic member (10) is a resin elastic member with an arcuate cross section.

8. The device according to claim 7, wherein the tactile switch (6) is provided inside the arc of the resin elastic member (10).

9. The device according to any one of claims 1 to 8, further comprising a communication means connected to the controller (3).

10. The device according to claim 9, wherein the communication means transmits a signal output from the controller (3) to a terminal (32).

11. The device according to any one of claims 1 to 10, wherein each tactile switch (6) is turned ON in response to a pressing force of 0,9 to 5 N.

## Patentansprüche

1. Vorrichtung zur Erfassung des Vorhandenseins/Nichtvorhandenseins einer Person in einem Bett, die umfasst:
eine Vielzahl von Schaltern (6), die in einer Lücke (25) zwischen der unteren Fläche einer Matratze (21) und einem Bodenmaterial (26) eines Betts (20) anordenbar sind, um die Matratze (24) darüber zu halten,
wobei die Schalter (6) jeweils einen Kontakt haben, durch eine angewendete Kraft EIN- und AUS-geschaltet werden, und
eine Steuerung (3), die das Vorhandensein oder Nichtvorhandensein der Person und/oder eine Änderung einer Körperposition der Person auf der Matratze (21) auf der Basis des EIN oder AUS der Schalter (6) erfasst,
**dadurch gekennzeichnet, dass**
die Schalter Berührungsschalter sind, von denen jeder einen Kontakt hat, der durch eine Kraft, die auf einen Kolben an seinem Spitzenende angewendet wird, und die Verformung einer Innenfeder EIN- und AUS-geschaltet wird, und
dass die Lücke durch ein elastisches Element zwischen der unteren Fläche der Matratze und dem Bodenmaterial des Betts ausgebildet wird, um die Matratze darüber zu halten, wobei das elastische Element die untere Fläche der Matratze nach oben vorspannt.

2. Vorrichtung nach Anspruch 1, wobei die Kolben jeweils flache Flächen auf ihren oberen Enden haben.

3. Vorrichtung nach Anspruch 1 oder 2, wobei das Bett (20) eine Vielzahl der elastischen Elemente (23) hat, welche die untere Fläche der Matratze (21) wenigstens in einem mittleren Teil des Bodenmaterials (26) des Betts (20) nach oben vorspannen, um die Matratze (21) darüber zu halten.

4. Vorrichtung nach einem der Ansprüche 1 bis 3, wobei die Berührungsschalter (6) an einem streifenförmigen Substrat (4) bereitgestellt sind, das mit einem Boden oder einem Balken angreift.

5. Vorrichtung nach Anspruch 1 oder 2, wobei das elastische Element (10) auf dem Bodenmaterial (26) des Betts (20) angeordnet ist und die untere Fläche der Matratze (21) nach oben vorspannt.

6. Vorrichtung nach Anspruch 1 oder 2, wobei das elastische Element (10) an der unteren Fläche der Matratze (21) befestigt ist und die untere Fläche der Matratze (21) nach oben vorspannt.

7. Vorrichtung nach Anspruch 5 oder 6, wobei das elastische Element (10) ein elastisches Harzelement mit einem bogenförmigen Querschnitt ist.

8. Vorrichtung nach Anspruch 7, wobei der Berührungsschalter (6) innerhalb des Bogens des elastischen Harzelements (10) bereitgestellt ist.

9. Vorrichtung nach einem der Ansprüche 1 bis 8, die ferner ein Kommunikationsmittel umfasst, das mit der Steuerung (3) verbunden ist.

10. Vorrichtung nach Anspruch 9, wobei das Kommunikationsmittel ein Signal, das von der Steuerung (3) ausgegeben wird, an ein Endgerät (32) überträgt.

11. Vorrichtung nach einem der Ansprüche 1 bis 10, wobei jeder Berührungsschalter (6) ansprechend auf eine Druckkraft von 0,9 bis 5 N EINgeschaltet wird.

## Revendications

1. Dispositif permettant de détecter la présence/l'absence d'une personne dans un lit, comportant :
une pluralité d'interrupteurs (6) pouvant être disposés dans un interstice (25) entre la surface inférieure d'un matelas (21) et un matériau de plancher (26) d'un lit (20) destiné à maintenir le matelas (21) sur celui-ci,
les interrupteurs (6), ayant chacun un contact, sont activés et désactivés par une force appliquée, et
un dispositif de commande (3) qui détecte la présence ou l'absence de la personne et/ou le changement de position du corps de la personne sur le matelas (21) sur la base de l'activation ou de la désactivation des interrupteurs (6),
**caractérisé en ce que**
lesdits interrupteurs sont des interrupteurs tactiles, chacun ayant un contact qui est activé et désactivé par une force appliquée sur un bouton-poussoir au niveau d'une portion d'extrémité de pointe de celui-ci et par déformation d'un ressort interne, et
**en ce que** l'interstice est formé par un élément élastique entre la surface inférieure du matelas et le matériau de plancher du lit destiné à maintenir le matelas sur celui-ci, l'élément élastique poussant la surface inférieure du matelas vers le haut.

2. Dispositif selon la revendication 1, dans lequel les boutons-poussoirs disposent de surfaces plates au niveau de leurs extrémités supérieures.

3. Dispositif selon les revendications 1 ou 2, dans lequel le lit (20) présente une pluralité des éléments élastiques (23) poussant la surface inférieure du matelas (21) vers le haut au moins au niveau d'une partie centrale du matériau de plancher (26) du lit (20) destiné de maintenir le matelas (21) sur celui-ci.

4. Dispositif selon l'une quelconque des revendications 1 à 3, dans lequel les interrupteurs tactiles (6) sont prévus sur un substrat en forme de bande (4) venant en prise avec un plancher ou une poutre.

5. Dispositif selon les revendications 1 ou 2, dans lequel l'élément élastique (10) est disposé sur le matériau de plancher (26) du lit (20) et pousse la surface inférieure du matelas (21) vers le haut.

6. Dispositif selon les revendications 1 ou 2, dans lequel l'élément élastique (10) est attaché à la surface inférieure du matelas (21) et pousse la surface inférieure du matelas (21) vers le haut.

7. Dispositif selon les revendications 5 ou 6, dans lequel l'élément élastique (10) est un élément élastique en résine ayant une section transversale arquée.

8. Dispositif selon la revendication 7, dans lequel l'interrupteur tactile (6) est prévu à l'intérieur de l'arc de l'élément élastique en résine (10).

9. Dispositif selon l'une quelconque des revendications 1 à 8, comportant en outre un moyen de communication relié au dispositif de commande (3).

10. Dispositif selon la revendication 9, dans lequel le moyen de communication transmet une sortie de signal, du dispositif de commande (3) vers un terminal (32).

11. Dispositif selon l'une quelconque des revendications 1 à 10, dans lequel chaque interrupteur tactile (6) est activé en réponse à une force de pression comprise entre 0,9 N et 5 N.
